# EUROPEAN PATENT APPLICATION

(11) **EP 1 410 818 A1**
(43) Date of publication of application: **21.04.2004**
(21) Application number: 02425629.9
(22) Date of filing: 14.10.2002
(51) Int. Cl.: A61M 5/50, A61M 5/28

(54) **Vial for injection and relative safety device**

(71) Applicant: Restelli, Sergio, 00100 Rome (IT); Righi, Nardino, 20047 Brugherio (Milano) (IT); Rossi, Roberto, 20151 Milano (IT)
(72) Inventor: Restelli, Sergio, 00100 Rome (IT); Righi, Nardino, 20047 Brugherio (Milano) (IT); Rossi, Roberto, 20151 Milano (IT)
(74) Representative: Petruzziello, Aldo

(57) **Abstract**

A vial (4) for injection comprising an elastically deformable container (40) wherein a solution for injection is hermetically contained, and a male connector (41, 42) protruding forward from the container (40) to engage in a female connector (30, 28) of a needle-carrier (2) that bears a needle (50). The needle (5) has a first tip (50) which protrudes forward from the needle-carrier to engage in the patient's body and a second tip (51) disposed in the female connector (30) of the needle-carrier to perforate the end of the male connector (41) of the vial so that, by means of manual pressure on the container (40) of the vial, the solution contained therein can be injected through the needle (5) into the patient's body. A safety device suitable to be applied to said needle for a vial so as to render it unusable after the injection is also described.

## Description

The present invention refers to a vial or ampoule, such as, for example, a hermetic vial prefilled with any type of medicinal solution and the like, particularly suitable to be applied to a needle to perform an injection. The present injection also refers to a safety device particularly suitable to be applied to a needle for a vial according to the invention.

At present to perform an injection in a patient syringes consisting of a syringe body with a needle mounted in the head and a plunger slidable inside the syringe body are used. On retraction of the plunger, the solution for injection, which is generally contained in a glass vial, is drawn into the syringe body through the needle. Then, the injection is performed and by pushing on the plunger said solution is injected from the syringe body through the needle into the patient's body. It is evident that said operation is rather awkward, impractical and requires an excessive waste of time.

Prefilled syringes are known to the art, wherein to perform the injection the user must only push the plunger. However, even said syringes are excessively complex, not very versatile and not suitable for all types of medicinal solutions. Furthermore, in some cases the medicinal products that are in contact with the small steel cannula of the prefilled syringe can obstruct the inside hole of the needle.

The object of the present invention is to overcome the drawbacks of the prior art by providing a vial for injection, suitable to be applied to a needle for injection in a practical, easy and functional manner for the operator.

Another object of the present invention is to provide a safety device suitable to be applied to a needle for a vial according to the invention, so as to prevent reuse of the needle after the injection and at the same time to avoid the risk of accidental injuries and needle sticks.

Yet another object of the present invention is to provide a vial for injection and a relative safety device for a needle for a vial that are economical, simple to make and at the same time practical for the user.

These objects are achieved in accordance with the invention with the vial according to independent claim 1.

Advantageous embodiments of the invention are apparent from the dependent claims.

The vial for injection according to the invention comprises a substantially tubular body, wherein a medicinal solution for injection is hermetically contained. The body of the vial is made of an elastically deformable plastic material and has a male connector, suitable to be coupled with a female connector of a needle-carrier which supports an injection needle.

The injection needle is hollow on the inside and has a first tip which protrudes forward from the needle-carrier to perform the injection in the patient and a second tip which extends inside the female connector of the needle-carrier. In this manner, when the male connector of the vial is coupled inside the female connector of the needle-carrier, the second tip of the needle perforates the end of the male connector of the vial coming into communication with the chamber inside the vial wherein the solution for injection is contained. At this point the user exerts finger pressure on the body of the vial, causing an elastic deformation thereof and thus injection of the solution, through the needle, into the patient's body.

The structure of the vial according to the invention and the manufacturing process thereof allow injectable drugs to be inserted therein, ready for use and in single-dose disposable packages. Said vials afford the following advantages:
- Production of pharmaceutical specialities in single-dose disposable packages that are easier to use with respect to the common injectable drugs with conventional syringes.
- Definitive elimination of the problem of possible reuse of the disposable syringes used to inject the same drugs with the conventional system (advantage, particularly for developing countries, of eliminating the possibility of transmitting viral hepatitis, HIV infection, etc., by reusing the same syringe several times without any subsequent washing and sterilization.
- Appreciable reduction in costs of manufacturing and using drugs that can be packaged with this method, having simplified the manufacturing system and completely eliminated use of the disposable syringe.
- Possibility of producing single-dose vaccines that are truly disposable, for underdeveloped countries, particularly sought after by the World Health Organisation (WHO), which has always sought disposable syringes for vaccines which self-destruct after use, irrespective of the user's wishes.
- Considerably simplified production of a medicinal product, not autoclavable with superheated steam, inserted in a new disposable, single-dose container, similar to a prefilled syringe.
- Complete elimination of the problem of putting prefilled syringes with blocked cannulas onto the market.

According to a further aspect of the invention, a safety device can be applied to the needle-carrier for vials. Said safety device for a needle for a vial comprises a needle-carrier or vial-carrier supporting an injection needle and able to receive a vial containing a medicinal solution for injection. A guide sleeve is fixed integrally to said needle-carrier. A syringe body is mounted axially slidably on the guide sleeve so as to be able to pass from a retracted position of use, wherein the needle protrudes outward therefrom, to a forward position of safety, wherein it covers the needle.

A spring is interposed between the needle-carrier and the syringe body to urge the syringe body into its extracted position of safety. The spring is compressed when the syringe body is in its retracted position of use. Locking means are provided such as to lock the syringe releasably in its retracted position of use against the action of the spring.

The advantages of the invention, which provides an extremely economical injection system that is easy for the user to use, are evident. Moreover, said invention also provides a safety system for application to the needle for a vial. In fact, once the injection has been completed, the operator manually operates the locking means to release the syringe body and thus the spring pushes the syringe body into its forward position of safety wherein the needle remains covered, avoiding reuse of the needle and possible accidental needle sticks.

Further characteristics of the invention will be made clearer by the detailed description that follows, referring to a purely exemplary and therefore non-limiting embodiment thereof, illustrated in the appended drawings, in which:
Fig. 1 is a perspective exploded view, illustrating a version complete with a vial for injection provided with the safety device which can be applied to the needle for a vial according to the invention;
Figure 2 is an exploded axial sectional view, illustrating three main elements forming part of the safety device of Figure 1;
Figure 3 is an axial sectional view of the safety device for the vial needle of Figure 1 assembled, in position ready for use, wherein the needle cover cap is not shown and wherein the needle and vial are shown partially broken off;
Figure 4 is a side view illustrating the safety device for a needle for a vial in its safety position;
Figure 5 is an axial sectional view, taken along the sectional plane V-V of Figure 4.

The vial according to the invention, denoted as a whole with reference numeral 4 and the safety device for a needle for a vial, denoted as a whole with reference numeral 1, are described with the aid of the figures.

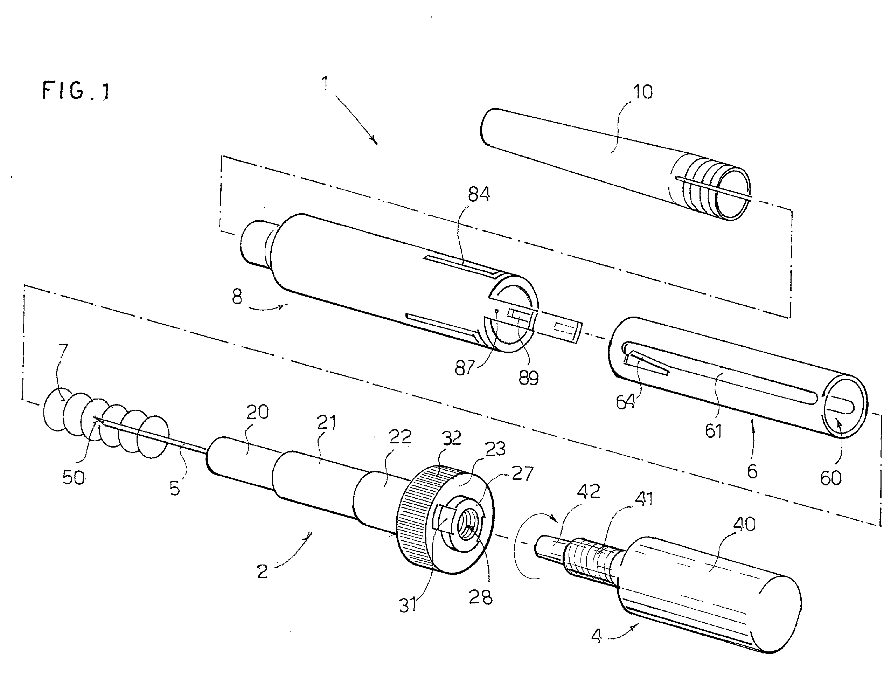

With specific reference for now to Figure 1, the safety device 1 for a needle for a vial is illustrated. The safety device 1 comprises:
- a needle-carrier or vial-carrier 2 bearing an injection needle 5 and suitable to receive a vial 4 containing the solution for injection,
- a guide sleeve 6 designed to be fixed integrally to the needle-carrier 2,
- a syringe body 8 mounted axially slidably on the guide sleeve 6,
- a spring 7 interposed between the needle-carrier 2 and the syringe body 8, and
- a cap 10 suitable to be mounted on the head of the syringe body 8 to cover the needle 5.

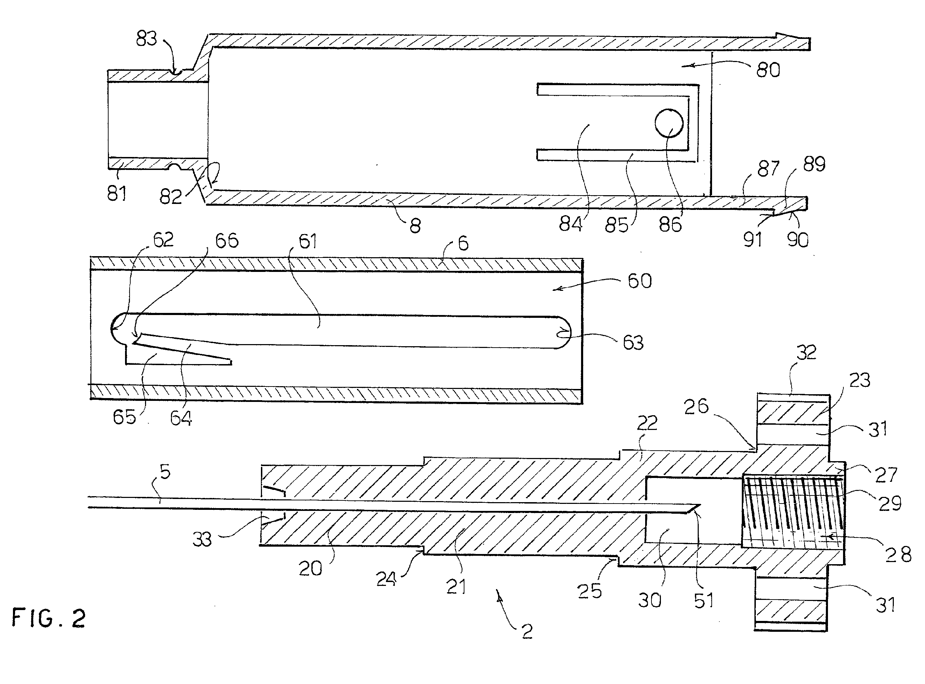

With reference also to Figure 2, the needle-carrier 2 consists of a substantially cylindrical body having a front portion 20, a central portion 21 with a greater diameter than the front portion 20 and a rear portion 22 with a greater diameter than the central portion 21. A cylindrical flange 23 having a greater diameter that the rear portion 22 is provided behind the rear portion 22.

In this manner, three annular abutment surfaces are defined, that is to say a first abutment surface 24 between the front portion 20 and the central portion 21, a second abutment surface 25 between the central portion 21 and the rear portion 22 and a third abutment surface 26 between the rear portion 22 and the flange 23.

The flange 23 has at the rear an annular protrusion 27 wherein a circular hole 28 having an inside thread 29 is formed. The hole 28 extends axially for the entire flange 23 to communicate with an axial chamber 30 provided in the rear portion 22 of the body of the needle-carrier.

Two through holes 31 disposed in diametrically opposite positions are formed in the peripheral part of the flange 23. Longitudinal knurls 32 are formed in the outer side surface of the cylindrical flange 23.

In the front end of the front portion 20 of the needle-carrier there is provided a flared hole 33 which continues with a through hole that passes axially through the entire body of the needle-carrier 2 to communicate with the chamber 30 in the rear portion 22.

The injection needle 5 proper is inserted and fixed in the axial hole of the needle-carrier 2, so that a first tip 50 of the needle protrudes forward from the body of the needle-carrier to be able to engage in the patient's skin and a second tip 51 of the needle is disposed in the chamber 30 in the rear part 22 of the body of the needle-carrier to be able to perforate the vial 4.

The vial 4 comprises a substantially tubular container 40, hollow on the inside, having a substantially elliptical cross section, wherein a medicinal solution for injection is contained. The container 40 of the vial is made of plastic and has a reduced thickness, so as to be able to be elastically deformed, by means of finger pressure by the user. The container 40 is preferably made of polyethylene.

The vial 4 has at the fore end a threaded male connector comprising a cylindrical tang 41 provided with an outside thread suitable to engage with the inside thread 29 of the axial hole of the flange 23 of the needle-carrier. The thread of the tang 41 of the vial is preferably quickfit, so that the vial can be screwed on to the needle-carrier with one turn around its own axis. The tang 41 of the vial ends with a cylindrical or frusto-conical front part 42 with a smaller diameter having a front end with reduced thickness to be able to be perforated by the second tip 51 of the needle. The threaded connector 41 of the vial is preferably made of hard plastic such as polypropylene.

The guide sleeve 6 is applied to the needle-carrier 2. The guide sleeve 6 has a cylindrical body hollow on the inside and having an axial cavity 60 open at the front and rear. The inside diameter of the sleeve 6 is equal to or slightly greater than the outside diameter of the rear portion 22 of the body of the needle-carrier 2, so that the sleeve 6 can be slipped over the needle-carrier 2.

Two longitudinal slots 61 disposed in diametrically opposite positions are formed in the side wall of the sleeve 6. Each longitudinal slot 61 is defined by a first rounded front abutment and end-of-stroke surface 62 and by a second rounded rear abutment and end-of-stroke surface 63.

A flexible tongue 64 is disposed obliquely in the front part of the longitudinal slot 61 so as to form a narrowing of the longitudinal slot 61. The tongue 64 has a rounded front end 66 opposed to the front abutment surface 62 of the longitudinal slot 61, so as to define a substantially circular locking seat.

The flexible tongue 64 is formed by means of a longitudinal cut 65 in the side wall of the sleeve 6. In this manner the tongue 64 can bend elastically in the longitudinal cut 65 widening the narrowing of the longitudinal slot.

The syringe body 8 is slidably mounted on the sleeve 6. The syringe body 8 comprises a cylindrical body hollow on the inside having an axial cavity 80 open at the front and rear. The inside diameter of the syringe body 8 is slightly greater than the outside diameter of the sleeve 6 so as to be able to be mounted thereon.

The syringe body 8 has at the front a head 81 having a smaller diameter than the body, so as to define an annular shoulder 82 on the inside. An annular seat 83 able to snap lock the needle cap 10 which is applied to the head is formed in the outer side surface of the head 81.

A first pair of flexible longitudinal tongues 84, disposed in diametrically opposite positions, is formed in the rear part of the side wall of the syringe body 8. Each longitudinal tongue 84 of the first pair of tongues is defined by a substantially upside down U-shaped cut, with the end of the tongue facing towards the rear part of the syringe body. In this manner each longitudinal tongue 84 can bend elastically radially outward and then return elastically to its initial position. A cylindrical pin 86 protruding radially inward is provided in the inside surface of the rear part of each tongue 84. The pin 86 has such a diameter as to be able to engage slidingly in the slot 61 of the sleeve.

A second pair of flexible longitudinal tongues 87 which protrude rearward from the syringe body 8 is provided in the rear end of the syringe body 8. The tongues 87 of the second pair of tongues are disposed in diametrically opposite positions and preferably spaced at an angular distance of 90° with respect to the first pair of tongues 84.

Each tongue 87 can bend elastically and radially outward and return elastically to the initial position. Furthermore each tongue 87 can be defined by two longitudinal parallel cuts formed at the rear end of the syringe body.

An outwardly protruding tooth 89 is provided in the outer surface of the rear part of each tongue. Each tooth 89 has a tapered outer surface 90 ending in a radial abutment surface 91.

The dimensions of the rear end tongues 87 of the syringe body are such as to allow insertion thereof into the through holes 31 formed in the flange 23 of the needle-carrier 2.

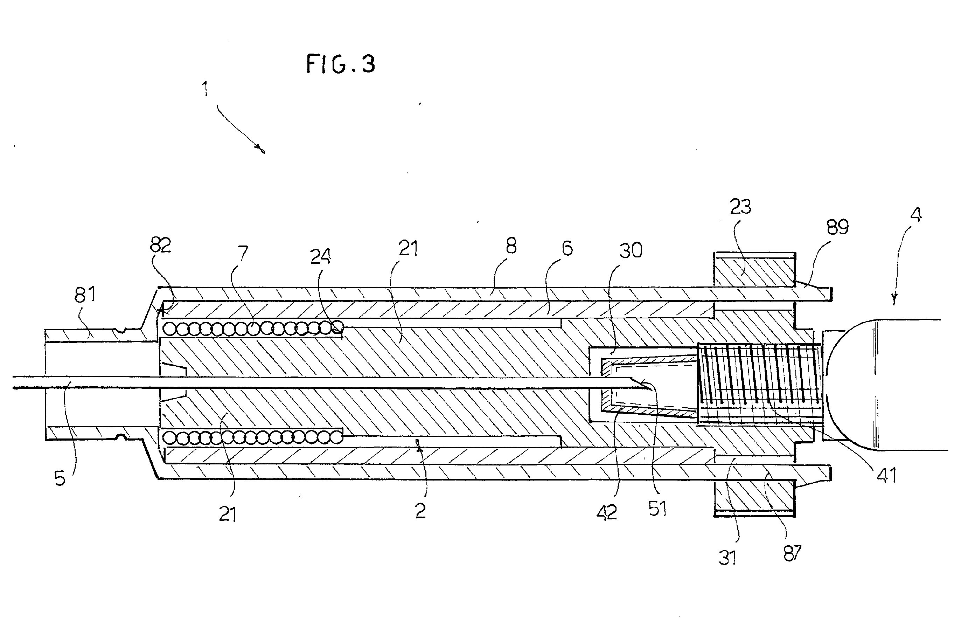

Assembly of the safety device 1 is illustrated hereunder by way of example, also with reference to Figure 3.

The guide sleeve 6 is slipped over the body of the needle-carrier 2, so that the rear end of the sleeve 6 abuts against the annular abutment surface 26 of the flange 23 of the needle-carrier. The sleeve 6 is then made integral with the needle-carrier, with suitable fixing means, within the reach of a person skilled in the art, such as for example gluing, welding, tongue and groove coupling or the like. Clearly the sleeve 6 can be made in a single piece, integral with the needle-carrier 2.

At this point the spring 7 is inserted between the inside surface of the sleeve 6 and the outside surface of the front part 20 of the needle-carrier 2, so that one end of the spring 7 abuts against the annular abutment surface 24 between the front part 20 and the central part 21 of the needle-carrier.

Lastly the syringe body 8 is slipped over the guide sleeve 6. During said operation the pins 86 of the first pair of tongues 84 of the syringe body are inserted and guided in the longitudinal guide slots 61 of the sleeve 6, until they abut against the respective abutment and end-of-stroke surfaces 63 of the guide slots 61.

The second pair of tongues 87, on the other hand, passes through the holes 31 in the flange 23 of the needle-carrier, so that the teeth 89 of the second pair of tongues 87 protrude rearward from the flange 23 and the abutment surface 91 of the teeth 89 abuts against the rear end of the flange 23. It should be noted that the tapered surface 90 of the teeth 89 facilitates insertion of the tongues 87 in the respective holes 31 of the flange 23.

When the syringe body is in its retracted rear end-of-stroke position, the spring 7 is compressed with one end thereof abutting against the abutment surface 24 of the needle-carrier 2 and the other end thereof abutting against the shoulder 82 of the syringe body. In said situation any axial movement of the syringe body 8 with respect to the sleeve 6 which is integral with the needle-carrier 2 is prevented.

In fact rearward movement of the syringe body is prevented by at least one of the constraints indicated below:
a) the shoulder 82 abuts against the front end of the sleeve 6
b) the pins 86 of the first pair of tongues abut against the rear abutment and end-of-stroke surface 63 of the sleeve 6, and
c) the rear end of the syringe body 8 abuts against the rear abutment surface 26 of the flange 23.

Forward movement of the syringe body 8, on the other hand, is prevented by the teeth 89 of the second pair of tongues 87 which engage in the rear end of the flange 23 of the needle-carrier.

At this point the needle 5 is ready for use. In fact the front tip 50 of the needle 5 protrudes forward out of the head 81 of the syringe body 8 and the threaded tang 41 of the vial 4 can be screwed into the threaded hole 28 of the flange 23 so that the front end of the head 42 of the vial is perforated by the rear tip 51 of the needle 5 disposed in the chamber 30 of the needle-carrier. Once the vial 4 has been applied to the needle-carrier, the user can insert the needle into the patient's body and press the body of the container 40 of the vial with his/her fingers, so as to cause the solution to pass from the chamber of the container of the vial to the patient's body through the needle.

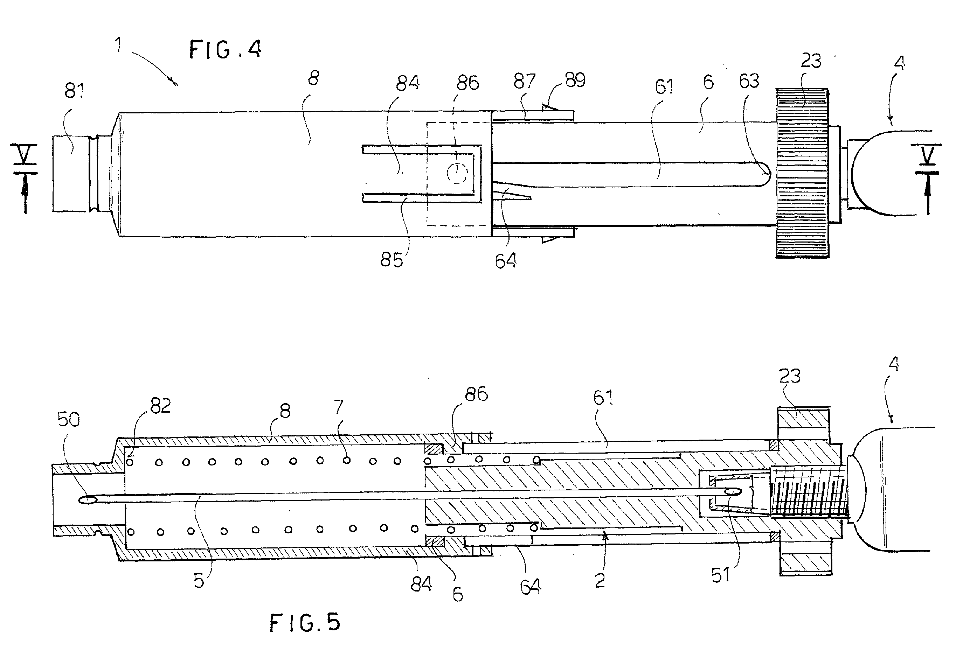

When the injection has been completed, the operator, with the fingers of one hand pushes the tongues 87 of the second pair of tongues inward, causing disengagement of the teeth 89 from the rear end of the flange 23. As a result, the syringe body 8 is no longer constrained in its axial forward movement. Thus, as shown in Figures 4 and 5, the spring 7, which was compressed, is released, pushing the syringe body 8 forward with respect to the sleeve 6 which is integral with the needle-carrier 2.

During said axial forward movement, the syringe body 8 is guided. In fact the pins 86 of the tongues 84 of the first pair of tongues of the syringe body are guided slidingly in the respective longitudinal guide slots 61 of the sleeve, until the pins 86 pass the narrowing of the slots 61 caused by the respective tongues 64 causing the tongues 64 to bend elastically in the respective cuts 65.

Thus, when the pins 86 abut against the respective front stopping and end-of-stroke surfaces 62 of the slots 61, the tongues 64 snap back elastically into their initial position. Thus, each pin 86 is blocked between the front end-of-stroke and abutment surface 62 of the slot 61 and the front abutment surface 66 of the elastic tongue 64. In this manner any axial movement of the syringe body 8 with respect to the sleeve 6 which is integral with the needle-carrier 2 is avoided.

In this situation the needle 5 remains in its initial position of safety completely covered by the syringe body 8, avoiding possible tampering with the safety device 1 and any accidental needle sticks.

In the present detailed description a vial 4 applied to a needle-carrier provided with a safety system has been illustrated, it being understood that the vial 4 according to the invention can also be applied to a needle-carrier 2 not having the safety system 1.

Numerous variations and modifications of detail within the reach of a person skilled in the art can be made to the present embodiment of the invention, without thereby departing from the scope of the invention as set forth in the appended claims.

## Claims

1. A vial (4) for injection comprising:
- an elastically deformable container (40) wherein a medicinal solution for injection is hermetically contained, and
- a male connector (41, 42) protruding forward from said container (40) to engage in a female connector (30, 28) of a needle-carrier (2) which bears a needle (5), said needle (5) having a first tip (50) which protrudes forward from the needle-carrier to engage in the patient's body and a second tip (51) disposed in said female connector (30) of the needle-carrier to perforate the end of said male connector (41) of the vial so that, by means of manual pressure on the container (40) of the vial, the solution contained therein can be injected through the needle (5) into the patient's body.

2. A vial (4) according to claim 1, **characterised in that** said connector of said vial comprises a threaded male connector (41) which engages in a threaded female connector (28, 29) of said needle-carrier.

3. A vial (4) according to claim 1 or 2, **characterised in that** said connector of said vial has at its end a cylindrical or frusto-conical portion (42) able to engage in said chamber (30) of the needle-carrier, said cylindrical or frusto-conical portion (42) of the vial having an end with reduced thickness to be able to be perforated by the second tip (51) of the needle.

4. A vial (4) according to any one of the preceding claims, **characterised in that** said container (40) of the vial is made of elastically deformable plastic material, such as polyethylene and said connector (41, 42) of the vial is made of rigid plastic material, such as polypropylene.

5. A vial according to any one of the preceding claims, **characterised in that** it comprises a safety device (1) that can be applied to said needle-carrier (2) to protect the needle (5) after the injection, said safety device (1) comprising:
- a guide sleeve (6) fixed integrally to the needle-carrier (2),
- a syringe body (8) mounted axially slidably on the guide sleeve (6) to pass from a retracted position of use, wherein the front tip (50) of the needle protrudes outward and forward therefrom, to a forward position of safety wherein the front tip (50) of the needle remains covered inside the syringe body (8),
- spring means (7) interposed between said needle-carrier (2) and said syringe body (8), said spring means (7) being under compression when the syringe body (8) is in its retracted position of use, and
- first locking means (87, 89) provided in said syringe body (8), said first locking means (87, 89) being able to engage releasably with said needle-carrier so as to lock said syringe body in its retracted position of use against the action of said spring means (7).

6. A vial according to claim 5, **characterised in that** said syringe body (8) of the safety device (1) comprises guide means (84, 86) able to engage with complementary guide means (61) provided in said sleeve (6) to guide axial sliding of said syringe body (8) on the sleeve, from the retracted position of use to the forward position of safety.

7. A vial according to claim 6, **characterised in that** said guide means of the syringe body (8) of the safety device (1) comprise a pair of radially inward protruding pins (86), and said complementary guide means of the sleeve (6) comprise a pair of longitudinal slots (61) able to receive said pins (86).

8. A vial according to claim 7, **characterised in that** said pair of pins (86) is disposed on a respective pair of longitudinal flexible tongues (84) formed by means of respective upside down U-shaped cuts in said syringe body.

9. A vial according to any one of claims 5 to 8, **characterised in that** it comprises second locking means (64) disposed in said sleeve (6) to lock said syringe body (8) when it is in its extracted position of safety.

10. A vial according to claim 9, when it is dependent upon claims 7 or 8, **characterised in that** said second locking means (64) are a pair of flexible tongues (64) disposed obliquely in the respective guide slots (61) of the sleeve (6) to snap lock said pins (86) of the syringe body, when the syringe body is in its forward position of safety.

11. A vial according to any one of claims 5 to 10 **characterised in that** said first locking means (87, 89) provided in said syringe body (8), comprise a pair of teeth (89) mounted on a pair of flexible tongues (87) formed in said syringe body, said teeth (89) being releasably engageable in said needle-carrier.

12. A vial according to claim 11, **characterised in that** said needle-carrier has at the rear a cylindrical flange (23) with a greater diameter than the body of the needle-carrier, there being formed in said flange (23), in a central position, a threaded hole (28) to receive the threaded head (41) of a vial (4) and there being formed in a peripheral position two through holes (31) able to receive said pair of tongues (87) which protrude rearward from the rear end of the syringe body (8).

13. A vial according to claims 6 and 11 **characterised in that** said tongues (84) supporting the guide pins (86) are disposed in diametrically opposite positions in proximity to the rear part of the syringe body (8) and said tongues (87) supporting the locking teeth (89) are disposed in diametrically opposite positions at the rear end of said syringe body (8), the guide tongues (84) and the locking tongues (87) being spaced apart from each other by an angle of about 90°.

14. A vial according to any one of claims 5 to 13, **characterised in that** said spring means comprise a spiral spring (7) having one end abutting against an annular shoulder (82) formed on the inside surface of the syringe body and the other end abutting against an annular abutment surface (24) formed on the outer surface of the body of the needle-carrier.

15. A safety device (1) for a needle for a vial **characterised in that** it comprises:
- a needle-carrier or vial-carrier (2) supporting an injection needle (5) provided with a front tip (50) for injection into the patient and a rear tip (51) to perforate a vial (4) containing a medicinal solution for injection, said vial (4) being able to be applied to said needle-carrier (2),
- a guide sleeve (6) fixed integrally to the needle-carrier (2),
- a syringe body (8) mounted axially slidably on the guide sleeve (6) to pass from a retracted position of use, wherein the front tip (50) of the needle protrudes outward and forward therefrom, to a forward position of safety, wherein the front tip (50) of the needle remains covered inside the syringe body (8),
- spring means (7) interposed between said needle-carrier (2) and said syringe body (8), said spring means (7) being under compression when the syringe body (8) is in its retracted position of use, and
- first locking means (87, 89) provided in said syringe body (8), said first locking means (87, 89) being able to engage releasably with said needle-carrier so as to lock said syringe body in its retracted position of use against the action of said spring means (7).
